# EUROPEAN PATENT APPLICATION

(11) **EP 2 662 082 A1**
(43) Date of publication of application: **13.11.2013**
(21) Application number: 13179744.1
(22) Date of filing: 14.11.2006
(51) Int. Cl.: A61K 31/436, A61P 35/00

(54) **Administration of mTOR inhibitors**

(30) Priority: 14.11.2005 US 736763 P
(62) Divisional of application: 06844354.8
(71) Applicant: Ariad Pharmaceuticals, Incorporated, Cambridge, MA 02139 (US)
(72) Inventor: Bedrosian, Camille, L., Belmont, MA Massachusetts 02478 (US)
(74) Representative: Duncan, Garreth Andrew

(57) **Abstract**

Disclosed are methods for treating a patient with an mTOR inhibitor such as AP23573, sirolimus, temsirolimus, everolimus, etc.

## Description

### TECHNICAL FIELD

This invention relates to the administration of an mTOR inhibitor to a patient in need thereof, especially to a cancer patient.

### BACKGROUND

Several mTOR inhibitors are currently under evaluation as single agents or in various combinations for the treatment of a variety of cancers. Those mTOR inhibitors include the rapamycin analogs, AP23573 (ARIAD Pharmaceuticals, inc.), everolimus (Novartis) and temsirolimus (Wyeth). Other mTOR inhibitors include, among others, sirolimus (rapamycin), and the additional analogs, ABT-578 and biolimus. While AP23573, everolimus and temsirolimus have all yielded positive results in human studies, mouth sores have been noted as a dose limiting toxicity.

Those mouth sores have previously been loosely termed "mucositis" in some cases. For a review of the pathobiology of mucositis, see Stephen T. Sonis, Nature Reviews | Cancer vol. 4, 277-284 (April 2004). See also, Rubenstein et al, Mucositis: Perspectives and Clinical Practice Guidelines Supplement to Cancer vol. 100, No. 9, pp. 2026 - 2046 (May 1, 2004). Actually, however, these mouth sores typically differ noticeably from the classic mucositis that frequently accompanies radiation therapy and other cancer therapies such as cytotoxic cancer chemotherapies. Nonetheless, these mouth sores can be debilitating and constitute a dose limiting toxicity for the use of the new mTOR inhibitors, severe enough for patients and caregivers to consider, and in some cases to implement, interruption or reduction of dosing with the mTOR inhibitor even though doing so can undercut the therapeutic impact of the mTOR inhibitor therapy.

This invention provides a new approach for the administration of an mTOR inhibitor, especially to cancer patients.

### SUMMARY OF THE INVENTION

Development of new drugs typically involves the use, where possible, of conventional formulation, delivery and dosing methodologies to achieve the simplest, most convenient and effective product forms and dosing schedules.

That is important for meeting commercial objectives for a new drug product and for optimal patient compliance. Often a pill, tablet, capsule or other oral dosage form that provides an effective, bioavailable form of the drug is of particular interest,

mTOR inhibitors are a recently expanded class of drugs useful for treating various cancers and other disorders. These include rapamycin (serolimus), AP23573, CCI779 (temserolimus), RAD001 (everolimus), which are mentioned above, as well as others. These drugs are typically administered in various dosages and on various schedules as tablets or as intravenous infusions.

It has now been found, however, that conventional, simple dosing schedules can be suboptimal for mTOR inhibitors. More particularly, therapeutic doses of an mTOR inhibitor administered daily, e.g., orally, typically for the treatment of any of a variety of cancers, is associated with the development of mouth sores in a subset of patients so treated, and of more severe mouth sores in a subset of those patients. However, decreasing the daily dose or adopting a less frequent dosing schedule risks a loss in effectiveness of the mTOR inhibitor regimen.

It has further been found that a continuous (i.e., week after week) 4-day or 5-day per week dosing schedule, e.g., QDx4 or QDx5 dosing, can yield a beneficial compromise between efficacy and the risk and severity of mouth sores. This can permit larger daily doses of an mTOR inhibitor and greater cumulative exposure to the drug than would be preferred on a 7-day/week schedule-without unduly increasing the risk of mouth sores, especially the risk of more severe grades of such mouth sores.

QDx4 and QDx5 dosing means that the mTOR inhibitor is administered to the patient in one or more doses per day for four or five consecutive days, respectively, followed by three or two days, respectively, without treatment with the mTOR inhibitor. The drug may be administered in one or more portions per day, e.g., twice a day ("bid") dosing. The administration of drug is maintained countinuously on a weekly basis, i.e., for at least two, and usually more than two, consecutive weeks (and thus without an intervening week without mTOR inhibitor).

The mTOR inhibitor may be any mTOR inhibitor, although AP23573, temserolimus and everolimus are currently of particular interest. A typical daily dose is from 2mg to 80 mg of drug, e.g., 5 - 60 mg, or 10 - 50 mg, or 10 - 40 mg, or 10 - 30 mg.

Oral administration of the drug, e.g., in tablet or capsule form, is of particular interest.

The QDx4 or QDx5 administration of this invention may further be supplemented with a loading dose of 2 - 300mg of an mTOR inhibitor given orally or parenterally on an intermittent basis, e.g., once per week, or once every second or third week. The mTOR inhibitor of the loading dose will usually be the same mTOR inhibitor given on the other days, but may be a different mTOR inhibitor. A loading dose which doubles or triples the normal daily dose is of particular current interest, as is administering the loading dose on the first day of the QDx4 or QDx5 cycle. For instance, in a regimen of 20 mg of the mTOR inhibitor daily for four (QDx4) or five days (QDx5), the optional loading dose may be an additional 20 or 40 mg of the mTOR inhibitor on day 1 of each week, or on day 1 every other week, or on day 1 of every third week, by way of example. The mTOR inhibitor may be AP23573, everolimus, rapamycin or another mTOR inhibitor. That loading dose may be administered orally or may be given parenterally, e.g., by i.v. infusion, and may be coordinated, in cases of combination therapies, with the administration of the other anticancer drug(s) of the combination.

The mTOR inhibitor may be administered as a monotherapy, or may be administered in coordination with the administration of one or more other drugs for treating the cancer or for alleviating the effects of the cancer or of any of the drugs given to the patient.

When the mTOR inhibitor is used as part of a combination regimen, dosages of each of the components of the combination are administered during a desired treatment period. The components of the combination may administered at the same time; either as a unitary dosage form containing both components, or as separate dosage units; the components of the combination can also be administered at different times during a treatment period, or one may be administered as a pretreatment for the other.

### DETAILED DESCRIPTION

The mTOR inhibitor may be administered by any pharmaceutically acceptable route, a variety of which are known for that class of drugs. Parenteral and, especially, oral administration are currently of particular interest. The mTOR inhibitors of greatest current interest are rapamycin analogs in which the hydroxyl group at position 43 is replaced, especially those analogs currently in clinical development for treating cancer, such as AP23573, Everolimus and Temsirollmus. These and other mTOR inhibitors are discussed in greater detail below.

Given the documented activity of mTOR inhibitors against a wide variety of cancers, the mTOR inhibitor therapy disclosed herein should be of interest for a correspondingly wide range of cancers. Those include among others prostate, endometrial, breast, ovarian, cervical, head and neck, small cell and non-small cell lung, pancreatic, kidney, brain, colorectal and bladder cancers as well as various sarcomas (including the various bone and soft tissue sarcomas), melanomas, multiple myeloma, B-cell lymphoma, mantle cell lymphoma, Non-Hodgkin's Lymphoma, CLL and CML, including, among others, cases which are advanced, recurrent, refractory to one or more other therapies and/or metastatic.

Moreover, additional drugs such as those described below may be given in conjunction with the mTOR inhibitor therapy of this invention.

The therapy disclosed herein constitutes a new method for treating various types of cancer and other diseases, with the objective of providing a desirable therapeutic window for achieving clinical benefit without incurring an unacceptable level of side effects.

As used herein, the term "treating" refers to the administration of an mTOR inhibitor and a second drug to a patient after the onset, or suspected onset, of a cancer. "Treating" includes the concepts of "alleviating", which refers to lessening the frequency of occurrence or recurrence, or the severity, of any symptoms or other ill effects related to a cancer and/or the side effects associated with cancer therapy. The term "treating" also encompasses the concept of "managing" which refers to reducing the severity of a particular disease or disorder in a patient or delaying its recurrence, e.g., lengthening the period of remission in a patient who had suffered from the disease.

The term "effective amount" or "effective dose", as used herein means the amount or dose of a substance that elicits a desirable biological or clinical response in a tissue or patient. For example, a desirable response may include one or more of the following: delaying or preventing the onset of a medical condition, disease or disorder; slowing down or stopping the progression, aggravation or worsening of the condition or symptoms of the condition; ameliorating the symptoms of the condition; and curing the condition-e.g., where the condition, disease, disorder or symptoms associated with cancer or to side effects of anti cancer therapy.

All combinations of method or process steps as used herein can be performed in any order, unless otherwise specified or clearly implied to the contrary by the context in which the referenced combination of steps is made.

All percentages, parts and ratios as used herein are by weight of the total composition, unless otherwise specified. All such weights as they pertain to listed ingredients are based on each respective ingredient per se and, therefore; do not include solvents or by-products that may be included in commercially available materials, unless otherwise specified.

The compositions and methods of the present invention can comprise additional or optional ingredients, components, or limitations described herein or otherwise useful in compositions and methods of the general type as described herein.

### 1. Rapamycin analogs ― mTOR inhibitors

Rapamycin is a macrolide produced by *Streptomyces hygroscopicus* and discovered in the 1970's. Rapamycin is a potent immunosuppressive agent and is used clinically to prevent rejection of transplanted organs. It has also been reported to have a wide range of interesting pharmacologic activities, making it and its derivatives of interest for a range of indications including the treatment and prevention of organ transplant rejection and autoimmune diseases, fungal infection, multiple sclerosis; rheumatoid arthritis, systemic lupus erythematosus [see e.g., U.S. Pat. No. 5,078,999], pulmonary inflammation [U.S. Pat. No. 5,080,899], insulin dependent diabetes mellitus [U.S. Pat. No. 5,321,009], skin disorders, such as psoriasis [U.S. Pat. No. 5,286,730], bowel,disorders [U.S. Pat. No. 5,286.731], smooth muscle cell proliferation and intimal thickening following vascular injury [U.S. Pat. Nos. 5,288,711 and 5,516,781], adult T-cell leukemia/lymphoma [European Patent Application 525,960 A1], ocular inflammation [U.S. Pat. No. 5,387,589], malignant carcinomas [U.S. Pat. No. 5,206,018], cardiac inflammatory disease [U.S. Pat. No. 5,496,832], and anemia [U.S. Pat. No. 5,561,138]. Use against cancer is of particular interest. See e.g. US Pat. application 2001/0010920. A number of derivatives of rapamycin, including AP23573 (ARIAD), CCI779 ("temsirolimus", Wyeth) and RAD001 ("Everolimus", Novartis) have yielded promising results in human studies against a variety of cancers. In addition, rapamycin and everolimus are used as immunosuppressants in organ transplant recipients. Rapamycin and a number of the C-43-modified rapamycin analogs, including among others AP23573, Biolimus and ABT-578 (Abbott), are being used, evaluated or developed for use on drug-eluting stents.

Because there is more than one accepted convention for numbering the positions of rapamycin, and derivatives thereof, the numbering convention used herein is depicted below: For reference, the R group for a number of compounds is set forth in the following table:

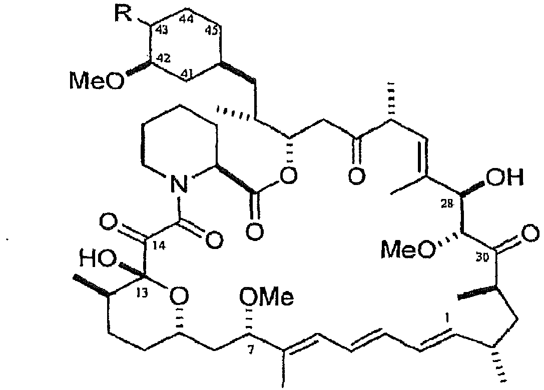

| **Compound** | -**R** |
|---|---|
| Rapamycin | -OH |
| AP23573 | -OP(O)(Me)₂ |
| Temsirolimus | -OC(O)C(CH₃)(CH₂OH) |
| Everolimus | -OCH₂CH₂OH |
| Biolimus | -OCH₂CH₂OEt |
| ABT-578 | -Tetrazole |

These compounds are non-limiting examples of potent mTOR inhibitors. Their administration to patients can lead to mouth sores, and such patients are the preferred subjects for the therapeutic compositions and methods of this invention. For additional information on AP23573, see US Patent No. 7,091,213. For recent references on temserolimus (CCI779), see WO 2004/026280, WO 2005/011688, WO 2005/070393, WO 2006/086172, and WO 2006/089312. For everolimus, see US Patent No. 6,384,046, WO 2002/066019, US 6,197,781, US 6,004,973 and references cited therein. Other mTOR inhibitors of interest include 42-desmethoxy derivatives of rapamycin and its various analogs, as disclosed, e.g., in WO 2006/095185 (in which such compounds are referred to as "39-desmethoxy" compounds based on their numbering system). The derivatives of rapamycin are of particular current interest in practicing this invention.

### 2. Formulation of mTOR inhibitors

A variety of oral and parenteral dosage forms are known for rapamycin and a number of rapamycin analogs. Some are currently in use in various treatment methods, monotherapies or otherwise. Those same dosage forms may likewise be used in the practice of the mTOR inhibitor therapy disclosed herein. Solid dosage forms are often preferred for oral administration and include among others conventional admixtures, solid dispersions and nanoparticles, typically in tablet, capsule, caplet, gel cap or other solid or partially solid form. Such formulations may optionally contain an enteric coating. Numerous materials and methods for such oral formulations are well known. A typical example of the use of conventional materials and methods to formulate an mTOR inhibitor is shown in US Patent Application US 2004/0077677 and Published International Patent Application WO04026280 (CCI-779). See also US patents US6197781, US6589536, US6555132, US5985321, US6565859 and US5932243. In addition to the foregoing nonlimiting examples of formulation technology, a wide variety of other methods and materials are also well known to those working in the field of macrolides like rapamycin and its derivatives. For additional background and examples of appropriate formulation technologies, see e.g., WO 03/064383.

In a preferred embodiment, the mTOR inhibitor is provided as an oral dosage form, such as a tablet. In the case of AP23573, for instance, the drug may prepared by a wet granulation process. The tablet may contain one or more cellulose polymers and one or more of an antioxidant, chelating agent, filler, binder, surfactant, desintegrant, lubricant, pH-modifying agent and the like. The wet granulation process may be performed with an aqueous or alcoholic, e.g., ethanolic solvent system. Other suitable alcohols include methanol, isopropanol, and the like. The solvent can also be a mixture of solvents, e.g. an alcoholic solvent and water.

It is currently of particular interest that the composition contain from 1 to 45%, from 2 to 35%, from 5 to 25%, or from 8 to 15% by weight of AP23573; from 1 to 50%, from 1 to 35%, from 1 to 15%, or from 2 to 15% by weight of cellulose polymer and from 0.01% to 3%, from 0.05% to 1 % or from 0.05% to 0.5% by weight of antioxidant. However, various embodiments may contain more, or less, of these components.

Acceptable antioxidants include, but are not limited to, citric acid, d,l-α-tocopherol, BHA, BHT, monothioglycerol, ascorbic acid, and propyl gallate. It is expected that the antioxidants of the formulations of this invention will be used in concentrations ranging from 0.001% to 3% wt/wt.

Chelating agents, and other materials capable of binding metal ions, such as ethylene diamine tetra acetic acid (EDTA) and its salts are capable of enhancing the stability of AP23573.

Typical cellulose polymers include, but are not limited to hydroxypropylmethylcellulose (HPMC), hydroxypropylmethyl cellulose phthalate, methyl cellulose (MC), hydroxyethyl cellulose, and hydroxypropyl cellulose (HPC).

Acceptable pH modifying agents include, but are not limited to citric acid, sodium citrate, dilute HCl, and other mild acids or bases capable of buffering a solution containing AP23573 to a pH in the range of about 4 to about 6. If present in the composition, the pH modifying agent is usually in amount of up to 1%.

Surfactants may be present in the formulation and include polysorbate 80, sodium lauryl sulfate, sodium dodecyl sulfate, salts of bile acids (taurocholate, glycocholate, cholate, deoxycholate, etc.) which may be combined with lecithin. Alternatively, ethoxylated vegetable oils, such as Cremophor EL, vitamin E tocopherol propylene glycol succinate (Vitamin ETGPS), polyoxyethylene-polyoxypropylene block copolymers, and poloxamers. If present in the composition, the surfactant is usually in amount of up to 20%, for example 1 to 15% by weight.

Binders, fillers, and disintegrants such as sucrose, lactose, microcrystalline cellulose, croscarmellose sodium, magnesium stearate, gum acacia, cholesterol, tragacanth, stearic acid, gelatin, casein, lecithin (phosphatides), carboxymethylcellulose calcium, carboxymethylcellulose sodium, methylcellulose, hydroxyethylcellulose, hydroxypropylcellulose, hydroxypropylmethyl-cellulose phthalate, noncrystalline cellulose, polyvinylpyrrolidone, cetostearyl alcohol, cetyl alcohol, cetyl esters wax, dextrates, dextrin, cyclodextrin, lactose, dextrose, glyceryl monooleate, glyceryl monostearate, glyceryl palmitostearate, polyoxyethylene alkyl ethers, polyethylene glycols, polyoxyethylene castor oil derivatives, polyoxyethylene stearates, and polyvinyl alcohol, and the like may also be incorporated into the formulation.

Any given formulation of this invention may contain multiple ingredients of each class of component. For example, a formulation containing an antioxidant may contain one or more antioxidants as the antioxidant component.

The tablet may further comprise a film-coat to control the release of the rapamycin analog. The tablet may be coated with a film-coat by spraying, dipping or by deposition. The film-coat typically includes a polymeric film-forming material such as copovidone (i.e a copolymer of polyvinylpyrrolidone and vinyl acetate), hydroxypropyl methylcellulose, hydroxypropylcellulose, and acrylate or methacrylate copolymers. Besides a film-forming polymer, the film-coat may further comprise a plasticizer, e.g. polyethylene glycol, triethyl citrate, a surfactant, e.g. a Tween.RTM type, an anti-foaming agent, e.g. Simethicone, and optionally a pigment, e.g. titanium dioxide or iron oxides. The film-coating may also comprise talc as anti-adhesive. The film coat usually accounts for less than about 5% by weight of the dosage form. In a preferred embodiment, the film-coating material comprises copovidone.

The film coating may also be an enteric layer comprising an enteric polymer, for delayed release of the rapamycin analog. An enteric layer is a coating of a substance (i.e a polymer) which is insoluble in the acid medium of the stomach but which is soluble at the higher pH encountered in the intestine. Such materials are used as film coatings on tablets to modify the release of a drug. Suitable enteric polymers are well known to those of skill in the art (WO 01/051031) and include, without limitation, methyl metacrylate polymers, methacrylic acid co-polymers, cellulose acetate phthalate, polyvinyacetate phthalate, hydroxypropyl methyl phthalate, and hydroxypropyl methyl cellulose phthalate. For instance, the enteric layer may comprise a methacrylic acid co-polymer such as Eudragit L100, Acryl-EZE or the like.

In addition to the foregoing non limiting examples of formulation technology, a wide variety of other methods and materials are also well known to those working in the field of macrolides like rapamycin and its derivatives. For additional background and examples of appropriate formulation technologies, see e.g., WO 03/064383 and US Published Patent Application 20050032825.

### 3. Method of Treatments

The mTOR inhibitor therapy disclosed herein encompasses methods of treating, preventing and/or managing various types of cancer while providing a desirable therapeutic window for achieving clinical benefit without incurring an unacceptable level of side effects.

Examples of cancers and cancer conditions that can be treated with the mTOR inhibitor therapy of this document include, but are not limited to, solid tumors such as sarcomas and carcinomas, lymphatic cancers and especially PTEN-deficient tumors (see e.g. Neshat et al, PNAS 98(18):10314 10319; Podsypanina et al, PNAS 98(18):01320-10325; Mills et al PNAS 98(18):10031-10033; Hidalgo et al, Oncogene (2000) 19, 6680-6686). PTEN-deficient tumors may be identified, using genotype analysis and/or in vitro culture and study of biopsied tumor samples. Non-limiting examples of cancers involving abnormalities in the phosphatidyl-inositol 3 kinase/Akt-mTOR pathway include, but are not limited to, glioma, lymphoma and tumors of the lung, bladder, ovary, endometrium, prostate or cervix which are associated with abnormal growth factor receptors (e.g., EGFR, PDGFR, IGF-R and IL-2); ovarian tumors which are associated with abnormalities in P13 3 kinase; melanoma and tumors of the breast, prostate or endometrium which are associated with abnormalities in PTEN; breast, gastric, ovarian, pancreatic, and prostate cancers associated with abnormalities with Akt; lymphoma, cancers of the breast or bladder and head and neck carcinoma associated with abnormalities In eIF-4E; mantle cell lymphoma, breast cancer and head and neck carcinomas associated with abnormalities in Cyclin D; and familial melanoma and pancreas carcinomas associated with abnormalities in P16.

By "solid tumors" are meant tumors and/or metastasis, such as brain and other nervous system tumors (e.g. tumors of the meninges, brain such as glioblastoma and astrocytomas, spinal cord and other parts of the central nervous system); head and/or neck cancer; breast tumors; excretory system tumors (e.g. kidney, renal, pelvis, bladder and other unspecified organs); gastrointestinal tract tumors (e.g. oesophagus, stomach, colon, small intestine, rectum, tumors involving the liver; gall bladder, pancreas and other parts of the digestive organs); oral cavity (lips, tongue, throat, mouth, tonsil, oropharynx, nasopharynx, and other sites); reproductive system tumors (e.g. vulva, cervix, uterus, ovary and other sites associated with female genital organs, penis, prostate, testis and other sites associated with male genital organs); respiratory tract tumors (e.g. nasal cavity, middle ear, sinuses, bronchus, lung and other sites); skeletal system tumors (e.g. bones, cartilage and other sites); skin tumors (e.g. malignant melanoma of the skin, non-melanoma skin cancer, carcinoma, sarcoma); and tumors involving other tissues including peripheral nerves, connective and soft tissue, eye and adnexa, thyroid, adrenal gland and other endocrine glands and related structures, secondary and unspecified malignant neoplasm of lymph nodes, secondary malignant neoplasm of respiratory and digestive systems and secondary malignant neoplasm of other sites. By "lymphatic cancers" are meant e.g. tumors of the blood and lymphatic system (multiple myeloma, lymphoid leukemia, myeloid leukemia, acute or chronic lymphocytic leukemia, monocytic leukemia, other leukemias of specified cell type, leukemia of unspecified cell type, other unspecified malignant neoplasms of lymphoid, haematopoietic and related tissues, for example T-cell lymphoma or cutaneous lymphoma).

Cancers that can be treated using this mTOR inhibitor therapy include among others cases which are refractory to treatment with other chemotherapeutics. The term *"refractory",* as used herein refers to a cancer (and/or metastases thereof), which shows no or only weak anti-proliferative response (e.g., no or only weak inhibition of tumor growth) after treatment with another chemotherapeutic agent. These are cancers that cannot be treated satisfactorily with other chemotherapeutics. Refractory cancers encompass not only (i) cancers where one or more chemotherapeutics have already failed during treatment of a patient, but also (ii) cancers that can be shown to be refractory by other means, e.g., biopsy and culture in the presence of chemotherapeutics.
The mTOR inhibitor therapy described herein is also applicable to the treatment of patients who have not been previously treated.

### 4. Administration and use

The mTOR inhibitor may be administered as described herein for treating, preventing and/or managing various types of cancers. The exact amount required will also vary from subject to subject, depending on age, and general condition of the subject, the severity of the cancer, the particular mTOR inhibitor, its mode of administration, and the like.

The specific effective dose level of mTOR inhibitor for any particular patient or organism will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed, and like factors well known in the medical arts.

In one embodiment of this invention, a 10mg dose of Rapamycin or AP23573, or Everolimus or Temsirolimus is administered to the patient every day for four to five consecutive days, followed by two or three days, respectively without treatment with the mTOR inhibitor.

In another embodiment of this invention, a 10mg dose of Rapamycin or AP23573, or Everolimus or Temsirolimus is administered to the patient, twice a day, every day for four to five consecutive days, followed by two or three days, respectively without treatment with the mTOR inhibitor.

It will be understood, however, that within the practice of the subject invention, the total daily usage of the compounds and compositions of the present invention will be decided by the attending physician within the scope of sound medical judgment.

### 5. Drug combinations

Treatment using an mTOR inhibitor in accordance with this invention may be provided in combination with one or more other cancer therapies, include surgery, radiotherapy (e.g.. gamma-radiation, neutron beam radiotherapy, electron beam radiotherapy, proton therapy, brachytherapy, and systemic radioactive isotopes, etc.), endocrine therapy, biologic response modifiers (e.g., interferons, interleukins, and tumor necrosis factor (TNF) to name a few), hyperthermia, cryotherapy, agents to attenuate any adverse effects (e.g., antiemetics), and other cancer chemotherapeutic drugs. The other agent(s) may be administered before, during or after administration of an mTOR inhibitor as provided by this invention and may be administered using a formulation, route of administration and dosing schedule the same or different from that provided herein for the mTOR inhibitor.

Alternatively or additionally, methods and compositions of the present invention can be employed together with other agents to attenuate any adverse effects (e.g., statins, pain medication, antiemetics, G-CSF, GM-CSF, etc.), and/or with other approved chemotherapeutic drugs. Such other drugs include but not limited to one or more of the following: an anti-cancer alkylating or intercalating agent (e.g., mechlorethamine, chlorambucil, Cyclophosphamide, Melphalan, and lfosfamide); antimetabolite (e.g., Methotrexate); purine antagonist or pyrimidine antagonist (e.g., 6-Mercaptopurine, 5-Fluorouracil, Cytarabile, capecitabine and Gemcitabine); spindle poison (e.g., Vinblastine, Vincristine, Vinorelbine and Paclitaxel); podophyllotoxin (e.g., Etoposide, Irinotecan, Topotecan); antibiotic (e.g., Doxorubicin, Bleomycin and Mitomycin); nitrosourea (e.g., Carmustine, Lomustine); inorganic ion (e.g., Cisplatin, Carboplatin, Oxaliplatin or oxiplatin); enzyme (e.g., Asparaginase); hormone (e.g., Tamoxifen, Leuprolide, Flutamide and Megestrol); proteasome inhibitor (such as Velcade, another proteasome inhibitor (see e.g., WO 02/096933) or another NF-kB inhibitor, including, e.g., an IkK inhibitor); other kinase inhibitors (e.g., an inhibitor of Src, BRC/Abi, kdr, fit3, aurora-2, glycogen synthase kinase 3 ("GSK-3"), EGF-R kinase (e.g., lressa, Tarceva, etc.), VEGF-R kinase, PDGF-R kinase, etc); an antibody, soluble receptor or other receptor antagonist against a receptor or hormone implicated in a cancer (including receptors such as EGFR, ErbB2, VEGFR, PDGFR, and IGF-R; and agents such as Herceptin (or other anti-Her2 antibody), Avastin, Erbitux, etc.); etc. For a more comprehensive discussion of updated cancer therapies see, http://www.nci.nih.gov/, a list of the FDA approved oncology drugs at http://www.fda.gov/cder/cancer/druglistframe.htm, and The Merck Manual, Seventeenth Ed. 1999, the entire contents of which are hereby incorporated by reference. Examples of other therapeutic agents include among others, Zyloprim, alemtuzumab, altretamine, amifostine, nestrozole, antibodies against prostate-specific membrane antigen (such as MLN-591, MLN591RL and MLN2704), arsenic trioxide, bexarotene, bleomycin, busulfan, capecitabine, Gliadel Wafer, celecoxib, chlorambucil, cisplatin-epinephrine gel, cladribine, cytarabine liposomal, daunorubicin liposomal, daunorubicin, daunomycin, dexrazoxane, docetaxel, doxorubicin, Elliott's B Solution, epirubicin, estramustine, etoposide phosphate, etoposide, exemestane, fludarabine, 5-FU, fulvestrant, gemcitabine, gemtuzumab-ozogamicin, goserelin acetate, hydroxyurea, idarubicin, idarubicin. idamycin, ifosfamide, imatinib mesylate, irinotecan (or other topoisomerase inhibitor, including antibodies such as MLN576 (XR11576)), letrozole, leucovorin, leucovorin levamisole,liposomal daunorubicin, melphalan, L-PAM, mesna, methotrexate, methoxsalen, mitomycin C, mitoxantrone, MLN518 or MLN608 (or other inhibitors of the flt-3 receptor tyrosine kinase, PDFG-R or c-kit), itoxantrone, paclitaxel, Pegademase, pentostatin, porfimer sodium, Rituximab (RITUXAN®), talc, tamoxifen, temozolamide, teniposide, VM-26, topotecan, toremifene, 2C4 (or other antibody which interferes with HER2-mediated signaling), tretinoin, ATRA, valrubicin, vinorelbine, or pamidronate, zoledronate or another bisphosphonate.

The mTOR inhibitor therapy of this invention can also be employed together with one or more combinations of cytotoxic agents as part of a treatment regimen, wherein the combination of cytotoxic agents is selected from: CHOPP (cyclophosphamide, doxorubicin, vincristine, prednisone, and procarbazine); CHOP (cyclophosphamide, doxorubicin, vincristine, and prednisone); COP (cyclophosphamide, vincristine, and prednisone); CAP-BOP (cyclophosphamide, doxorubicin, procarbazine, bleomycin, vincristine, and prednisone); m-BACOD (methotrexate, bleomycin, doxorubicin, cyclophosphamide, vincristine, dexamethasone, and leucovorin); ProMACE-MOPP (prednisone, methotrexate, doxorubicin, cyclophosphamide, etoposide, leucovorin, mechloethamine, vincristine, prednisone, and procarbazine); ProMACE-CytaBOM (prednisone, methotrexate, doxorubicin, cyclophosphamide, etoposide, leucovorin, cytarabine, bleomycin, and vincristine); MACOP-B (methotrexate, doxorubicin, cyclophosphamide, vincristine, prednisone, bleomycin, and leucovorin); MOPP (mechloethamine, vincristine, prednisone, and procarbazine); ABVD (adriamycin/doxorubicin, bleomycin, vinblastine, and dacarbazine); MOPP (mechloethamine, vincristine, prednisone and procarbazine) alternating with ABV (adriamycin/doxorubicin, bleomycin, and vinblastine); MOPP (mechloethamine, vincristine, prednisone, and procarbazine) alternating with ABVD (adriamycin/doxorubicin, bleomycin, vinblastine, and dacarbazine); ChlVPP (chlorambucil, vinblastine, procarbazine, and prednisone); IMVP-16 (ifosfamide, methotrexate, and etoposide); MIME (methyl-gag, ifosfamide, methotrexate, and etoposide); DHAP (dexamethasone, high-dose cytaribine, and cisplatin); ESHAP (etoposide, methylpredisolone, high-dose cytarabine, and cisplatin); CEPP(B) (cyclophosphamide, etoposide, procarbazine, prednisone, and bleomycin); CAMP (lomustine, mitoxantrone, cytarabine, and prednisone); CVP-1 (cyclophosphamide, vincristine, and prednisone), ESHOP (etoposide, methylpredisolone, high-dose cytarabine, vincristine and cisplatin); EPOCH (etoposide, vincristine, and doxorubicin for 96 hours with bolus doses of cyclophosphamide and oral prednisone), ICE (ifosfamide, cyclophosphamide, and etoposide), CEPP(B) (cyclophosphamide, etoposide, procarbazine, prednisone, and bleomycin), CHOP-B (cyclophosphamide, doxorubicin, vincristine, prednisone, and bleomycin), CEPP-B (cyclophosphamide, etoposide, procarbazine, and bleomycin), and P/DOCE (epirubicin or doxorubicin, vincristine, cyclophosphamide, and prednisone).

The following examples contain additional information, exemplification and guidance which can be adapted to the practice of this invention in its various embodiments and the equivalents thereof. The examples are intended to help illustrate the invention, and are not intended to, nor should they be construed to, limit its scope in any way. Indeed, various modification of the invention, and many further embodiments thereof, in addition to those shown and described herein, will be apparent to those skilled in the art upon review of this document, including the examples which follow and the references to the scientific and patent literature cited herein . Such modifications and variations, including design choices in selecting, preparing, formulating and administering the mTOR inhibitor or the second drug of this invention, etc. are intended to be encompassed by the scope of the invention and of the appended claims.

The various pharmaceutical compositions of this invention may be prepared by any known or otherwise effective technique, suitable for making and formulating pharmaceutical dosage forms. Many such methods are described in the pharmaceutical arts or are otherwise well known to those skilled in their respective formulation arts.

The following example further describes and demonstrates certain specific embodiments within the scope of the invention. The example is given solely for the purpose of illustration and is not to be construed as a limitation of the present invention, as many variations thereof are of course possible and will be apparent to the practitioner without departing from the spirit and scope of the invention. All exemplified amounts are weight percentages based upon the total weight of the composition, unless otherwise specified.

### EXAMPLES

### Example 1: Oral Formulation AP23573

The following procedure was used to prepare a tablet containing 10 mg of AP23573 and containing the following components. The tablets are coated with two different coatings - a film-coated tablet for immediate release and an enteric-coated tablet for delayed release. The composition of the core tablet is shown in the following table. Core tablets are film-coated and may be used as such, or may be enteric-coated.

| **Component** | **Weight Percent** |
|---|---|
| AP23573 | 8.00% |
| Butylated Hydroxytoluene | 0.08% |
| Hydroxy Propyl Cellulose | 8% |
| Lactose Monohydrate | 50.57% |
| Microcrystalline Cellulose | 30.85% |
| Croscarmellose Sodium | 2.00% |
| Magnesium Stereate | 0.50% |
| Dehydrated Alcohol (Ethanol)* | - |

| | |
|---|---|
| *Use in processing but does not appear in final product | |

Hydroxypropyl Cellulose, Lactose Monohydrate, Microcrystalline Cellulose, and half of the Croscarmellose Sodium, were mixed in a high shear granulator. The AP23573 and Butylated Hydroxytoluene (BHT) were dissolved in Dehydrated Alcohol, USP, mixing not less than 45 minutes. The solution of AP23573 and BHT was added to the granulator and mixed to a wet mass for approximately 3 minutes.

The granulated mixture was dried in a fluid bed dryer at 45-55°C for 60-90 minutes, after which the dried mixture was passed through a mill fitted with a 0.045-inch screen opening to remove oversized material. The milled granulated mixture was then blended with Magnesium Stearate, NF and the remaining half of the Croscarmellose Sodium, NF.

The milled, granulated material was pressed into tablets using a tablet press set up with 6 mm round concave tooling. The press was adjusted as required for a target tablet weight of 125.0 mg, hardness of 5.5 kp, friability no more than 1%, and disintegration time less than 10 minutes.

### Film Coating

A film coating may be prepared according to following procedure using the following components. The tablets are added to a coating pan and are coated with a solution of Copovidone in Dehydrated Alcohol, USP (20:80 w/w), maintaining a product temperature of 20-35°C, until a weight gain of 5% is achieved. The pan is then cooled and the film-coated tablets allowed to dry. Film-coated tablets may be packaged as such, or may be enteric coated.

### Enteric Coating

An enteric coating may be prepared according to following procedure using the following components.

| **Film Coating** | **Percent of Suspension** |
|---|---|
| Methacrylic Acid Copolymer | 11.03% |
| Triethyl Citrate | 2.16% |
| Talc | 2.81% |
| Dehydrated Alcohol (Ethanol)* | 84.00% |

| | |
|---|---|
| *Use in processing but not for retention in final product | |

For enteric coating, the tablets are placed in a coating pan and coated with a suspension of Methacrylic Acid Copolymer, NF, Triethyl Citrate, NF, and Talc in Dehydrated Alcohol, USP, maintaining a product temperature of 20-35°C, until a weight gain of 8% is achieved. The pan is then cooled, and the enteric-coated tablets allowed to dry.

### Examp/e 2: A phase I dose escalation trial of oral AP23573 in patients with refractory or advanced malignancies

**Background:** In phase I clinical trials with an intravenous (IV) formulation ot the mTOR inhibitor, AP23573, the drug was was well-tolerated and active in a broad range of cancers. This trial is undertaken to assess the safety, tolerability and maximum tolerated dose (MTD) of an orally administered dosage form of AP23573. Secondary objectives include characterization of the pharmacokinetic (PK) and pharmacodynamic (PD) profiles of AP23573, as well as antitumor activity.

**Methods:** Eligible patients (in cohorts of at least 3 patients) initially are randomized at the same flat, fixed starting dose of 20 mg/day into one of three 28-day dosing schedules, i.e. once daily continuous dosing for 4 days every week (QDx4), 21 of 28 days (QDx21), or all 28 days (QDx28). The subsequent dose level is determined based on review of safety and tolerability during Cycle 1 and enrollment for each schedule proceeds independently. Antitumor activity is assessed every 2 cycles. Whole blood is collected for PK assessment and peripheral blood mononuclear cells collected for PD assessment of the levels of phosphorylated 4E-BP1, a downstream target of mTOR.

**Results:** To date, a total of 24 patients (11 females and 13 males) with a median age of 53.5 years (range 25-82) have received AP23573 on three schedules at doses ranging from 10 to 30 mg/day. For the QDx4 20 mg/day cohort, no dose-limiting toxicity (DLT) was reported and QDx4 30, 60 and 70mg/day were then explored. For both the QDx28 and QDx21 cohorts, severe stomatitis and fatigue were reported as DLTs at 20mg/day. Adverse events related to AP23573 during Cycle 1 in at least 2 patients include: mucositis, fatigue, rash, diarrhea, anorexia, and nausea. These events were mild or moderate in severity, reversible, and consistent with the toxicities of IV AP23573. Preliminary PK analysis indicates a median Cₘₐₓ of 98.5 ng/mL (range 77-163) for the three schedules (20 mg/day, Day 1 of Cycle 1). Further PK analysis is ongoing. PD analysis demonstrates that phosphorylated 4E-BP1 levels are reduced by >80% 1 day after dosing and remain reduced during the dosing period, indicating potent and durable mTOR inhibition similar to that observed in with AP23573 IV. Antitumor activity was observed in two patients evaluated to date: a patient with metastatic breast carcinoma in the qdx4, 20 mg/day cohort (22% decrease), and a patient with metastatic renal cell carcinoma in the qdx21,20mg/day cohort (21 % decrease). A third patient, with soft tissue sarcoma in the QDx4, 20 mg/day cohort has stable disease after 2 cycles.

**Conclusions:** The mTOR inhibitor, AP23573, can be safely administered orally using several continuous and intermittent dosing schedules, achieves blood concentrations that portend activity with IV administration, potently inhibits mTOR, and has demonstrated early evidence of antitumor activity, with reported DLTs for patients on the QDx28 and QDx21 schedules, but no DLTs for patients on the QDx4 20 and 30mg/day schedule.

However, for the QDx4 60 and 70 mg/day cohort, side effects were reported. The maximum tolerated daily dose was determined to be 50mg/day for the QDx4 schedule as opposed to 10 mg/day for QDx21 schedule and 15 mg/day for QDx28 schedule.

### Example 3: Additional studies

Additional clinical studies of AP23573 were conducted using a QDx5 dosing schedule for the delivery of 30, 40 or 50 mg of AP23573/day. A set of patients receiving the 30 mg and 50 mg doses were also given a loading dose on the first day of each week, which doubled the dose that day to 60 and 100 mg, respectively.

**Methods:** In a Phase 1/2a trial of single-agent oral AP23573, 17 patients, 9 of whom are sarcoma patients, were studied at the chosen dose and schedule (40 mg QDx5). In addition, of 7 patients who began dosing at 50 mg QDx5, 4 patients (2 of whom are sarcoma patients) underwent dose reduction to 40 mg. Twenty-one patients, therefore, received 40 mg QDx5, of whom 11 are sarcoma patients.

In another QDx5 study in which the dose on day 1 of each week was doubled (to comprise a loading dose), 7 patients (6 sarcoma patients) received doses of 60, 30, 30, 30 and 30 mg over each week (i.e., 30 mg QDx5, with the dose on day 1 doubled). Five other patients (2 sarcoma patients) received 50 mg QDx5, with the dose on day 1 doubled to 100mg. In that study, the latter dose level was considered to exceed the maximum tolerated dose for those patients.

Overall, 36 patients were dosed on a QDx5 schedule, of whom 12 received loading doses on day 1 of each week.

Results: These studies demonstrated evidence of anti-tumor activity as assessed by patients with Clinical Benefit Response (CBR). Seven patients of the 26 evaluable patients achieved a CBR. Six of them are sarcoma patients, including patients with osteosarcoma, leiomyosarcoma and other soft-tissue sarcomas. 43% of the thus far evaluable sarcoma patients were CBRs on these QDx5 schedules. The CBR patietns have been progression-free and receiving treatment from at least 4 months to 7 months to date.

**Conclusions:** the oral dose schedule of 40 mg QDx5 provided a good balance of potential therapeutic benefit against the risk of side-effects that were generally mild or moderate and manageable. The 40 mg QDx5 regimen every week provided a cumulative dose exposure of 800 mg AP23573 over 4 weeks, compared to the 4-week exposure level of 720 mg for a 30 mg QDx5 regimen (with a doubled day-1 dose each week), the 4-week exposure level of 315 mg for 15 mg QDx21, and the 4-week exposure level of 280 mg for a daily dose of 10 mg (i.e., 10 mg QDx28).

From these results it was concluded that the QDx5 dosing regimen, optionally augmented by the loading doses, achieves a preferred balance of maximizing drug exposure while limiting side-effects to a reasonable level, where maximizing drug exposure should translate to maximizing anti-tumor activity.

The present disclosure will now be further defined in the following numbered paragraphs:
1. A method for administering an mTOR inhibitor to a patient in need thereof which comprises administering the mTOR inhibitor to the patient each day for four or five consecutive days per week.
2. The method of paragraph 1, wherein the mTOR inhibitor is sirolimus, temsirolimus, everolimus or AP23573.
3. The method of paragraph 1, wherein the total daily dose of the mTOR inhibitor is 2-80 mg.
4. The method of paragraph 1, wherein the administration of the mTOR inhibitor is divided into two or more portions per day.
5. The method of paragraph 1, wherein the mTOR inhibitor is administered orally.
6. The method of paragraph 1, which further comprises administering an additional 2-300 mg of an mTOR inhibitor once per week.
7. The method of paragraph 1, which further comprises administering one or more other drugs in addition to the mTOR inhibitor.
8. The method of paragraph 1, wherein the patient is a cancer patient.
9. The method of paragraph 8, wherein the cancer is a sarcoma, lymphoma, or leukemia or a cancer of the bladder, colon, brain, breast, head and neck, endometrium, lung, ovary, pancreas or prostate.

## Claims

1. An mTOR inhibitor for use as a medicament, wherein the mTOR inhibitor is administered to a patient each day for four or five consecutive days per week.

2. The mTOR inhibitor for use of Claim 1, wherein the mTOR inhibitor is sirolimus, temsirolimus, everolimus or ridaforolimus.

3. The mTOR inhibitor for use of claim 1, wherein the total daily dose of the mTOR inhibitor is 2 - 80 mg.

4. The mTOR inhibitor for use of claim 1, wherein the administration of the mTOR inhibitor is divided into two or more portions per day.

5. The mTOR inhibitor for use of claim 1, wherein the mTOR inhibitor is administered orally.

6. The mTOR inhibitor for use of claim 1, which use further comprises administering an additional 2 - 300 mg of an mTOR inhibitor once per week.

7. The mTOR inhibitor for use of Claim 1, which use further comprises administering one or more other drugs in addition to the mTOR inhibitor.

8. The mTOR inhibitor for use of Claim 1, wherein the patient is a cancer patient.

9. The mTOR inhibitor for use of claim 8, wherein the cancer is a sarcoma, lymphoma, or leukemia or a cancer of the bladder, colon, brain, breast, head and neck, endometrium, lung, ovary, pancreas or prostate.
